(19) Europäisches Patentamt
European Patent Office
Office européen des brevets

(11) Veröffentlichungsnummer: **0 316 733 A1**

(12) **EUROPÄISCHE PATENTANMELDUNG**

(21) Anmeldenummer: **88118631.6**

(51) Int. Cl.⁴: **C07D 231/52 , A01N 43/56**

(22) Anmeldetag: **09.11.88**

(30) Priorität: **17.11.87 DE 3738963**

(43) Veröffentlichungstag der Anmeldung: **24.05.89 Patentblatt 89/21**

(84) Benannte Vertragsstaaten: **BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Jensen-Korte, Uta, Dr.**
**Geibelstrasse 9**
**D-4000 Düsseldorf 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Strang, Harry, Dr.**
**Unterdorfstrasse 6A**
**D-4000 Düsseldorf 13(DE)**

(54) **3-Aminopyrazolin-5-one.**

(57) Die Erfindung betrifft neue 3-Aminopyrazolin-5-one der Formel (I),

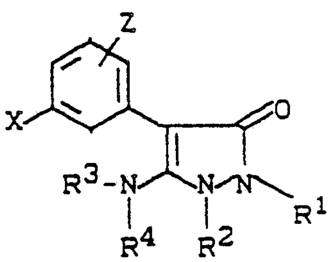

(I)

in welcher
$R^1$ für Alkyl, Alkenyl, Halogenalkyl, Halogenalkenyl, Alkoxyalkyl, Alkylthioalkyl, Cycloalkyl, für gegebenenfalls substituiertes Aralkyl oder für gegebenenfalls substituiertes Aryl steht,
$R^2$ für Wasserstoff oder Alkyl steht,
$R^3$ für Wasserstoff oder Alkyl steht,
$R^4$ für Wasserstoff, Alkyl, Alkenyl, Alkinyl, Alkoxyalkyl, Alkylthioalkyl, Alkoxycarbonylalkyl oder Alkanoyl steht oder
$R^3$ und $R^4$ gemeinsam für einen jeweils zweifach verknüpften Alkylen- oder Alkenylenrest stehen,
X für Halogen, Alkyl, Alkoxy, Alkylthio, Halogenalkyl, Halogenalkoxy oder Halogenalkylthio steht und
Z für Wasserstoff, Halogen, Alkyl, Alkoxy, Alkylthio, Halogenalkyl, Halogenalkoxy oder Halogenalkylthio steht,

EP 0 316 733 A1

sowie deren Säureadditionssalze, mehrere Verfahren sowie neue Zwischenprodukte zu ihrer Herstellung und ihre Verwendung als Herbizide.

## 3-Aminopyrazolin-5-one

Die Erfindung betrifft neue 3-Aminopyrazolin-5-one, mehrere Verfahren zu ihrer Herstellung und ihre Verwendung als Herbizide.

Es ist bekannt, daß bestimmte Amino-substituierte 5-Ring-Heterocyclen, beispielsweise bestimmte 2-Amino-thiazolin-4-one, wie die Verbindung 2-Amino-5-methyl-3-(3-trifluormethylphenyl)-5H-thiazolin-4-on herbizide Eigenschaften besitzen (vgl. z. B. US 4 596 595).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue 3-Aminopyrazolin-5-one der allgemeinen Formel (I),

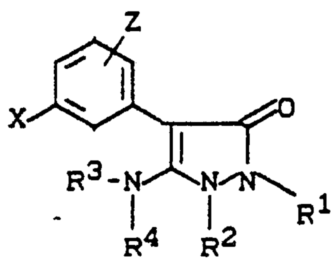

(I)

in welcher

$R^1$ für Alkyl, Alkenyl, Halogenalkyl, Halogenalkenyl, Alkoxyalkyl, Alkylthioalkyl, Cycloalkyl, für gegebenenfalls substituiertes Aralkyl oder für gegebenenfalls substituiertes Aryl steht,

$R^2$ für Wasserstoff oder Alkyl steht,

$R^3$ für Wasserstoff oder Alkyl steht,

$R^4$ für Wasserstoff, Alkyl, Alkenyl, Alkinyl, Alkoxyalkyl, Alkylthioalkyl, Alkoxycarbonylalkyl oder Alkanoyl steht oder

$R^3$ und $R^4$ gemeinsam für einen jeweils zweifach verknüpften Alkylen- oder Alkenylenrest stehen,

X für Halogen, Alkyl, Alkoxy, Alkylthio, Halogenalkyl, Halogenalkoxy oder Halogenalkylthio steht und

Z für Wasserstoff, Halogen, Alkyl, Alkoxy, Alkylthio, Halogenalkyl, Halogenalkoxy oder Halogenalkylthio steht,

sowie deren Säureadditionssalze gefunden.

Für den Fall, daß einer oder mehrere der Substituenten $R^2$, $R^3$ oder $R^4$ für Wasserstoff stehen, können die Verbindungen der Formel (I) in Form verschiedener tautomerer Strukturen vorliegen:

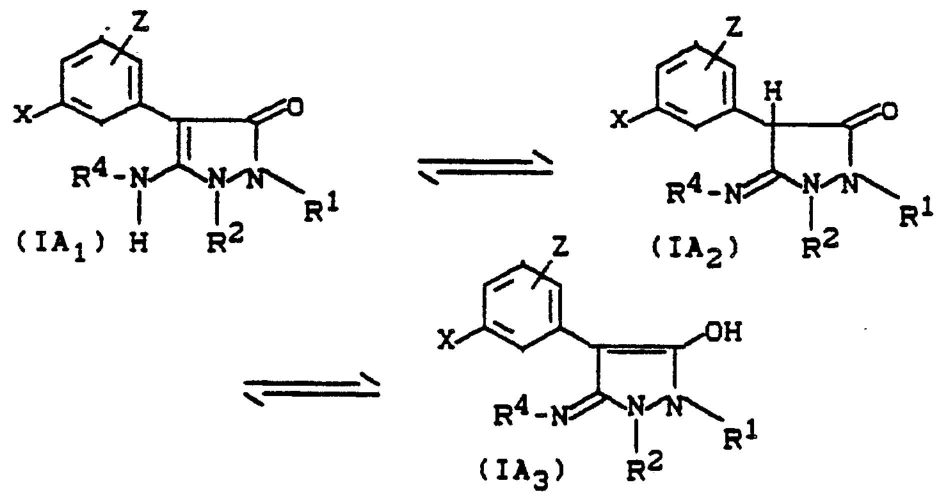

(IA1) (IA2) (IA3)

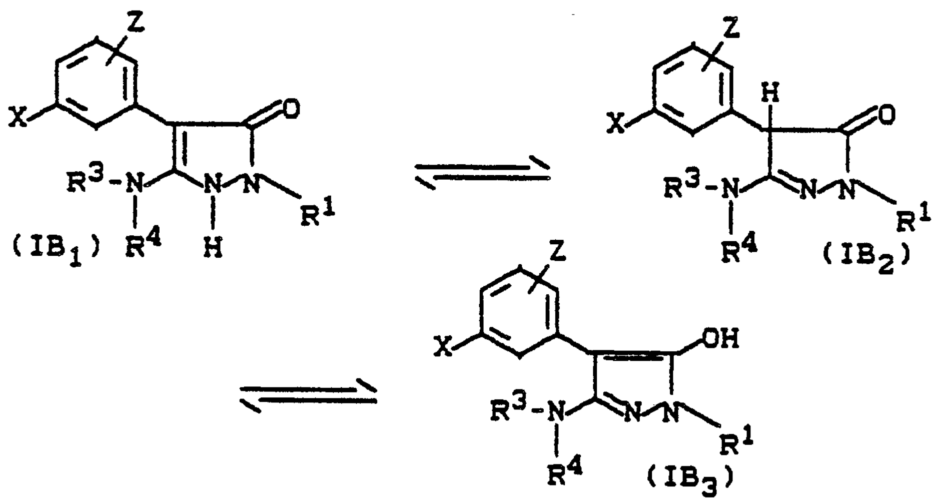

(IB1) (IB2) (IB3)

Die Erfindung umfaßt alle möglichen tautomeren Strukturen.

Weiterhin wurde gefunden, daß man die neuen 3-Aminopyrazolin-5-one der allgemeinen Formel (I),

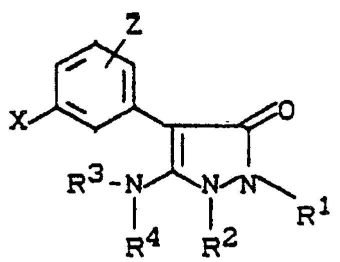

(I)

in welcher

$R^1$ für Alkyl, Alkenyl, Halogenalkyl, Halogenalkenyl, Alkoxyalkyl, Alkylthioalkyl, Cycloalkyl, für gegebenenfalls substituiertes Aralkyl oder für gegebenenfalls substituiertes Aryl steht,

$R^2$ für Wasserstoff oder Alkyl steht,

$R^3$ für Wasserstoff oder Alkyl steht,

$R^4$ für Wasserstoff, Alkyl, Alkenyl, Alkinyl, Alkoxyalkyl, Alkylthioalkyl, Alkoxycarbonylalkyl oder Alkanoyl steht oder

$R^3$ und $R^4$ gemeinsam für einen jeweils zweifach verknüpften Alkylen- oder Alkenylenrest stehen,

X für Halogen, Alkyl, Alkoxy, Alkylthio, Halogenalkyl, Halogenalkoxy oder Halogenalkylthio steht und

Z für Wasserstoff, Halogen, Alkyl, Alkoxy, Alkylthio, Halogenalkyl, Halogenalkoxy oder Halogenalkylthio steht,

sowie deren Säureadditionssalze nach einem der im folgenden beschriebenen Verfahren erhält:

(a) Man erhält 3-Aminopyrazolin-5-one der Formel (Ia),

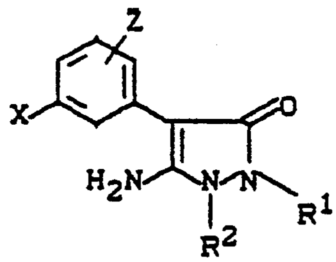

(Ia)

in welcher
$R^1$, $R^2$, X and Z die oben angegebene Bedeutung haben,
wenn man Iminomalonesterderivate der Formel (II),

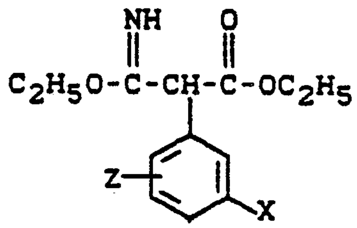

x HCl (II)

in welcher
X und Z die oben angegebene Bedeutung haben,
mit Hydrazinen der Formel (III),

$R^1$ - NH - NH - $R^2$ (III)

in welcher
$R^1$ und $R^2$ die oben angegebene Bedeutung haben,
oder deren Säureadditionssalzen gegebenenfalls in Gegenwart eines Verdünnungsmittels und gegebenenfalls in Gegenwart eines Reaktionshilfsmittels umsetzt;

(b) man erhält 3-Aminopyrazolin-5-one der Formel (Ib),

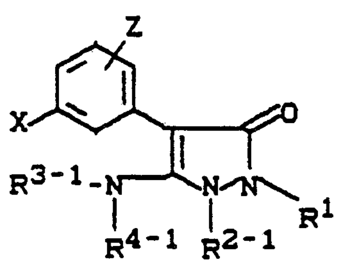

(Ib)

in welcher mindestens einer der Reste
$R^{2-1}$, $R^{3-1}$ oder $R^{4-1}$ für Alkyl steht und gleichzeitig die beiden nicht betroffenen Reste jeweils für Wasserstoff oder Alkyl stehen und
$R^1$, X and Z die oben angegebene Bedeutung haben,
wenn man die nach Verfahren (a) erhältlichen 3-Aminopyrazolin-5-one der Formel (Ia),

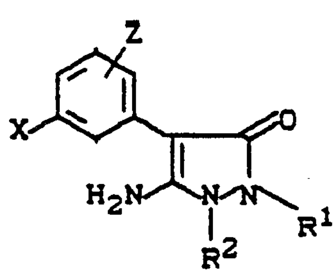

(Ia)

in welcher

$R^1$, $R^2$, X and Z die oben angegebene Bedeutung haben,
mit Alkylierungsmitteln der Formel (IVa),
$R^5$ - $E^1$ (IVa)
in welcher
$R^5$ für Alkyl steht und
$E^1$ für eine elektronenanziehende Abgangsgruppe steht,
gegebenenfalls in Gegenwart eines Verdünnungsmittels und gegebenenfalls in Gegenwart eines Reaktionshilfsmittels sowie gegebenenfalls in Gegenwart eines Phasentransferkatalysators umsetzt;

(c) man erhält 3-Aminopyrazolin-5-one der Formel (Ic),

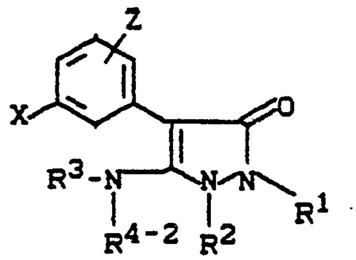

(Ic)

in welcher
$R^{4-2}$ für Alkenyl, Alkinyl, Alkoxyalkyl, Alkylthioalkyl oder Alkoxycarbonylalkyl steht und
$R^1$, $R^2$, $R^3$, X and Z die oben angegebene Bedeutung haben,
wenn man die nach Verfahren (a) und (b) erhältlichen 3-Aminopyrazolin-5-one der Formel (Iz),

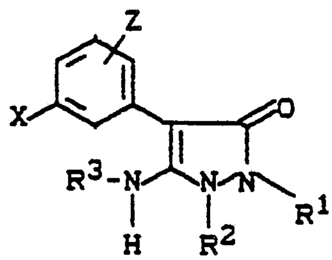

(Iz)

in welcher
$R^1$, $R^2$, $R^3$, X und Z die oben angegebene Bedeutung haben,
mit Alkylierungsmitteln der Formel (IVb),
$R^{4-2}$ - $E^2$ (IVb)
in welcher
$R^{4-2}$ die oben angegebene Bedeutung hat und
$E^2$ für eine elektronenanziehende Abgangsgruppe steht,
gegebenenfalls in Gegenwart eines Verdünnungsmittels und gegebenenfalls in Gegenwart eines Reaktionshilfsmittels sowie gegebenenfalls in Gegenwart eines Phasentransferkatalysators umsetzt;

(d) man erhält 3-Aminopyrazolin-5-one der Formel (Id),

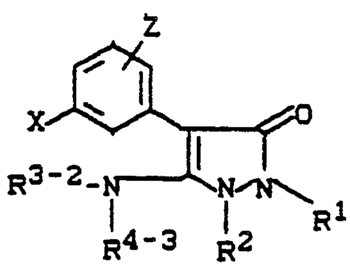

(Id)

in welcher
$R^{3-2}$ und $R^{4-3}$ gemeinsam für einen jeweils zweifach verknüpften Alkylen- oder Alkenylenrest stehen und
$R^1$, $R^2$, X and Z die oben angegebene Bedeutung haben,
wenn man die nach Verfahren (a) erhältlichen 3-Aminopyrazolin-5-one der Formel (Ia),

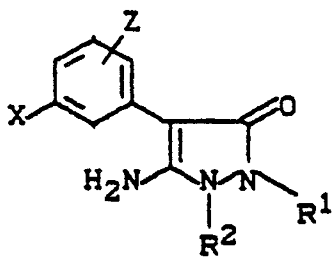

(Ia)

in welcher
$R^1$, $R^2$, X und Z die oben angegebene Bedeutung haben,
mit Alkylierungsmitteln der Formel (IVc),
$E^3$ - A - $E^3$ (IVc)
in welcher
A für einen Alkylen- oder Alkenylenrest steht und
$E^3$ für eine elektronenanziehende Abgangsgruppe steht,
gegebenenfalls in Gegenwart eines Verdünnungsmittels und gegebenenfalls in Gegenwart eines Reaktionshilfsmittels sowie gegebenenfalls in Gegenwart eines Phasentransferkatalysators umsetzt;

(e) man erhält 3-Aminopyrazolin-5-one der Formel (Ie),

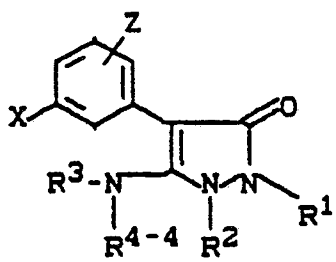

(Ie)

in welcher
$R^{4-4}$ für Alkanoyl steht und
$R^1$, $R^2$, $R^3$, X and Z die oben angegebene Bedeutung haben,
wenn man die nach Verfahren (a) oder (b) erhältlichen 3-Aminopyrazolin-5-one der Formel (Iz),

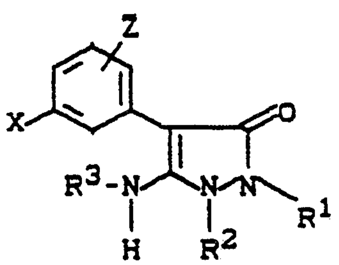

(Iz)

in welcher
$R^1$, $R^2$, $R^3$, X und Z die oben angegebene Bedeutung haben,
mit Acylierungsmitteln der Formel (V),
$R^{4-4}$ - $E^4$ (V)
in welcher
$R^{4-4}$ die oben angegebene Bedeutung hat und
$E^4$ für eine elektronenanziehende Abgangsgruppe steht,
gegebenenfalls in Gegenwart eines Verdünnungsmittels und gegebenenfalls in Gegenwart eines Reaktionshilfsmittels umsetzt;
und gegebenenfalls anschließend eine Säure addiert.

Schließlich wurde gefunden, daß die neuen 3-Aminopyrazolin-5-one der allgemeinen Formel (I) eine sehr gute herbizide Wirksamkeit besitzen.

Überraschenderweise zeigen die erfindungsgemäßen 3-Aminopyrazolin-5-one der allgemeinen Formel (I) eine erheblich bessere herbizide Wirksamkeit als die aus dem Stand der Technik bekannten Amino-substituierten 5-Ring-Heterocyclen wie beispielsweise das 2-Amino-5-methyl-3-(3-trifluormethylphenyl)-5H-thiazolin-4-on, welche chemisch und wirkungsmäßig naheliegende Verbindungen sind.

Die erfindungsgemäßen 3-Aminopyrazolin-5-one sind durch die Formel (I) allgemein definiert. Bevorzugt sind Verbindungen der Formel (I), bei welchen

$R^1$ für jeweils geradkettiges oder verzweigtes Alkyl mit 1 bis 6 Kohlenstoffatomen, Alkenyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und 1 bis 5 gleichen oder verschiedenen Halogenatomen, Alkoxyalkyl mit jeweils 1 bis 4 Kohlenstoffatomen in den einzelnen Alkylteilen oder Alkylthioalkyl mit jeweils 1 bis 4 Kohlenstoffatomen in den einzelnen Alkylteilen, für Cycloalkyl mit 3 bis 7 Kohlenstoffatomen oder für jeweils im Arylteil gegebenenfalls einfach oder mehrfach, gleich oder verschieden substituiertes Aralkyl mit 1 bis 4 Kohlenstoffatomen im Alkylteil und 6 bis 10 Kohlenstoffatomen im Arylteil oder Aryl mit 6 bis 10 Kohlenstoffatomen steht, wobei als Arylsubstituenten jeweils in Frage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl,

$R^2$ für Wasserstoff oder für geradkettiges oder verzweigtes Alkyl mit 1 bis 4 Kohlenstoffatomen steht,

$R^3$ für Wasserstoff oder für geradkettiges oder verzweigtes Alkyl mit 1 bis 4 Kohlenstoffatomen steht,

$R^4$ für Wasserstoff oder für jeweils geradkettiges oder verzweigtes Alkyl mit 1 bis 4 Kohlenstoffatomen, Alkenyl mit 3 bis 6 Kohlenstoffatomen, Alkinyl mit 3 bis 6 Kohlenstoffatomen, Alkoxyalkyl, Alkylthioalkyl oder Alkoxycarbonylalkyl mit jeweils 1 bis 4 Kohlenstoffatomen in den einzelnen Alkylteilen oder Alkanoyl mit 1 bis 5 Kohlenstoffatomen steht oder

$R^3$ und $R^4$ gemeinsam für einen jeweils zweifach verknüpften Alkylen- oder Alkenylenrest mit jeweils 2 bis 5 Kohlenstoffatomen stehen,

X für Fluor, Chlor, Brom, Iod, für jeweils geradkettiges oder verzweigtes Alkyl, Alkoxy oder Alkylthio mit jeweils 1 bis 4 Kohlenstoffatomen oder für jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen steht und

Z für Wasserstoff, Fluor, Chlor, Brom, Iod, für jeweils geradkettiges oder verzweigtes Alkyl, Alkoxy oder Alkylthio mit jeweils 1 bis 4 Kohlenstoffatomen oder für jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen steht.

Besonders bevorzugt sind Verbindungen der Formel (I), bei welchen,

$R^1$ für Methyl, Ethyl, n- oder i-Propyl, n-, i-, s-oder t-Butyl, für Allyl, n- oder i-Butenyl, für Halogenalkyl mit 1 bis 2 Kohlenstoffatomen und 1 bis 5 gleichen oder verschiedenen Halogenatomen, insbesondere Fluor, Chlor oder Brom, für jeweils gegebenenfalls einfach oder zweifach durch Chlor substituiertes Allyl, n-Butenyl oder i-Butenyl, für Methoxymethyl, Methylthiomethyl, für Cyclopropyl, Cyclopentyl, Cyclohexyl oder für jeweils gegebenenfalls ein- bis dreifach gleich oder verschieden substituiertes Phenyl, Naphthyl, Indenyl, Benzyl oder Phenylethyl steht, wobei als Arylsubstituenten jeweils in Frage kommen: Fluor, Chlor, Brom, Cyano, Nitro, Methyl, Ethyl, n- oder i-Propyl, n-, i-, s-oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder gegebenenfalls ein- bis dreifach, gleich oder verschieden durch Fluor, Chlor, Brom, Methyl und/oder Ethyl substituiertes Phenyl,

$R^2$ für Wasserstoff, Methyl oder Ethyl steht,

$R^3$ für Wasserstoff, Methyl, Ethyl, n- oder i-Propyl, n-, i-, s- oder t-Butyl steht,

$R^4$ für Wasserstoff, Methyl, Ethyl, n- oder i-Propyl, n-, i-, s- oder t-Butyl, Allyl, Propargyl, Methoxymethyl, Methylthiomethyl, Methoxycarbonylmethyl, Ethoxycarbonylmethyl, für Acetyl oder für Propionyl steht oder

$R^3$ und $R^4$ gemeinsam für dem Stickstoffatom, an welches sie gebunden sind für einen der Reste

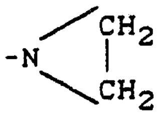 ; 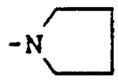 ; 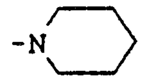 ; 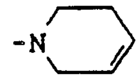 oder

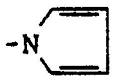 stehen,

X für Fluor, Chlor, Brom, Methyl, Methoxy, Methylthio, Trifluormethyl, Trifluormethoxy oder Trifluormethylthio steht und

Z für Wasserstoff, Fluor, Chlor, Brom, Methyl, Methoxy, Methylthio, Trifluormethyl, Trifluormethoxy oder Trifluormethylthio steht.

Bevorzugte erfindungsgemäße Verbindungen sind auch Additionsprodukte aus Säuren und denjenigen 3-Amino-pyrazolin-5-one der Formel (I), in denen die Substituenten $R^1$, $R^2$, $R^3$, $R^4$, X und Z die Bedeutungen haben, die bereits vorzugsweise für diese Substituenten genannt wurden.

Zu den Säuren die addiert werden können, gehören vorzugsweise Halogenwasserstoffsäuren, wie z. B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z. B. Essigsäure, Maleinsäure, Bernsteinsäure, Fumarsäure, Weinsäure, Zitronensäure, Salicylsäure, Sorbinsäure und Milchsäure, Sulfonsäuren, wie z. B. p-Toluolsulfonsäure und 1,5-Naphthalindisulfonsäure.

Ganz besonders bevorzugt sind Verbindungen der Formel (I), bei welchen

$R^1$ für Methyl, Ethyl, n- oder i-Propyl oder n-Butyl, für Allyl, für Cyclohexyl oder für jeweils gegebenenfalls ein- bis dreifach gleich oder verschieden substituiertes Benzyl oder Phenyl steht, wobei als Substituenten in Frage kommen: Fluor, Chlor, Brom, Nitro, Methyl, Methoxy, Ethyl oder Trifluormethyl,

$R^2$ für Methyl steht,

$R^3$ für Wasserstoff, Methyl, Ethyl, n- oder i-Propyl steht,

$R^4$ für Wasserstoff, Methyl, Ethyl, n- oder i-Propyl, für Allyl oder Propargyl steht,

X für Fluor, Chlor oder Trifluormethyl steht und

Z für Wasserstoff oder Fluor steht.

Im einzelnen seien außer den bei den Herstellungsbeispielen genannten Verbindungen die folgenden 3-Amino-pyrazolin-5-one der allgemeinen Formel (I) genannt:

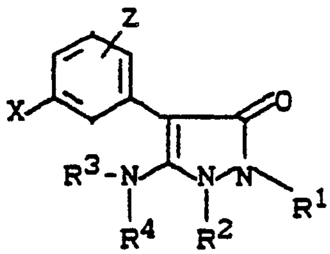

(I)

Tabelle 1

| $R^1$ | $R^2$ | $R^3$ | $R^4$ | 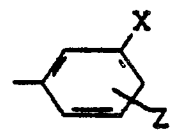 |
|---|---|---|---|---|
| 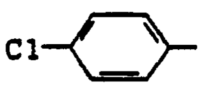 | $CH_3$ | H | $CH_3$ | 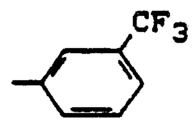 |
| 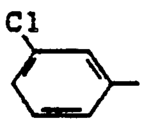 | $CH_3$ | H | $-CH_2-CH=CH_2$ | 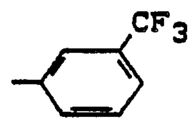 |
| 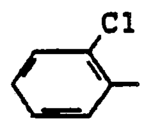 | $CH_3$ | $CH_3$ | $CH_3$ | 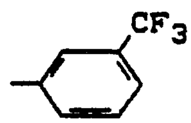 |
| 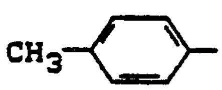 | $CH_3$ | H | $CH_3$ | 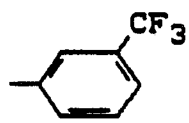 |

<u>Tabelle 1</u> Fortsetzung

| $R^1$ | $R^2$ | $R^3$ | $R^4$ | 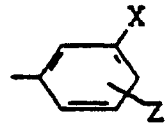 |
|---|---|---|---|---|
| 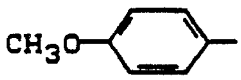 | $CH_3$ | H | $CH_3$ | 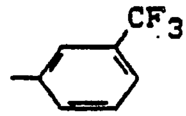 |
| 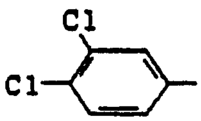 | $CH_3$ | H | $CH_3$ | 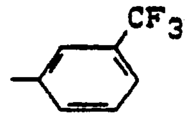 |
| 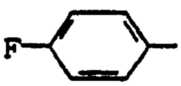 | $CH_3$ | H | $CH_3$ | 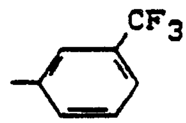 |
| 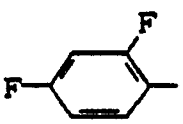 | $CH_3$ | H | $CH_3$ | 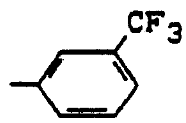 |
| 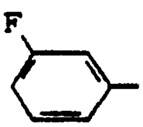 | $CH_3$ | H | $CH_3$ | 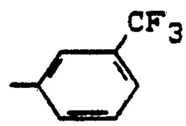 |
| 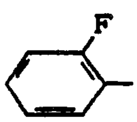 | $CH_3$ | H | $CH_3$ | 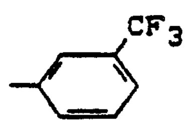 |
| 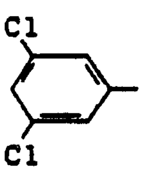 | $CH_3$ | H | $CH_3$ | 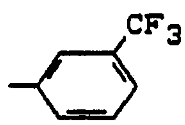 |
| 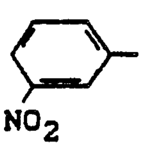 | $CH_3$ | H | $CH_3$ | 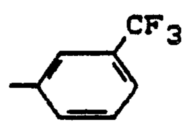 |

Tabelle 1 Fortsetzung

| $R^1$ | $R^2$ | $R^3$ | $R^4$ | 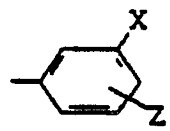 |
|---|---|---|---|---|
| 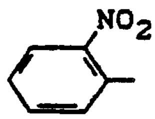 | $CH_3$ | H | $CH_3$ | 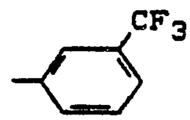 |
| 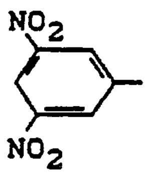 | $CH_3$ | H | $CH_3$ | 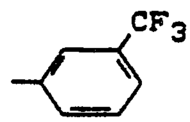 |
| 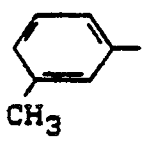 | $CH_3$ | H | $CH_3$ | 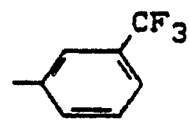 |
| 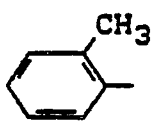 | $CH_3$ | H | $CH_3$ | 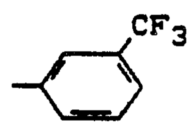 |
| 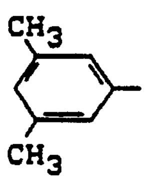 | $CH_3$ | H | $CH_3$ | 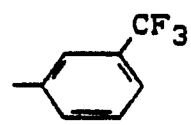 |
| 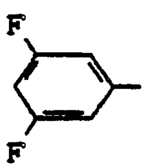 | $CH_3$ | H | $CH_3$ | 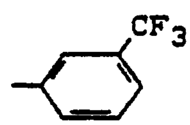 |
| 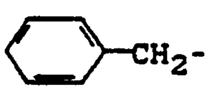 | $CH_3$ | H | $CH_3$ | 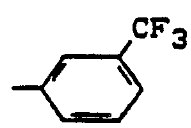 |

Tabelle 1 Fortsetzung

| $R^1$ | $R^2$ | $R^3$ | $R^4$ | 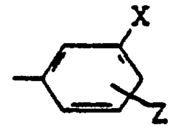 |
|---|---|---|---|---|
| 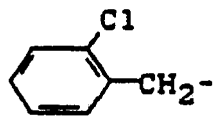 | $CH_3$ | H | $CH_3$ | 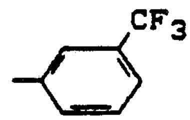 |
| 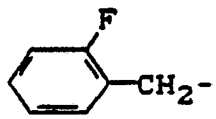 | $CH_3$ | H | $CH_3$ | 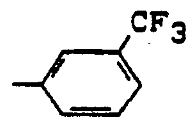 |
| 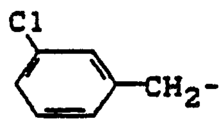 | $CH_3$ | H | $CH_3$ | 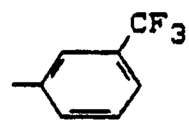 |
| 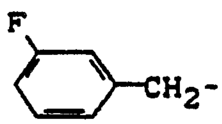 | $CH_3$ | H | $CH_3$ | 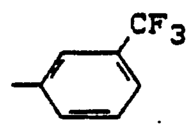 |
| 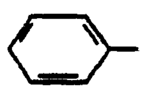 | $CH_3$ | H | $C_2H_5$ | 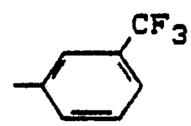 |
| $C_2H_5$ | $CH_3$ | H | $CH_3$ | 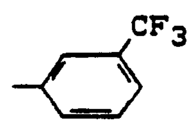 |
| 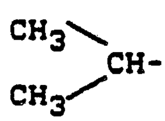 | $CH_3$ | H | $CH_3$ | 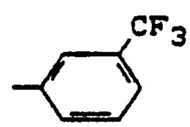 |
| $CH_3-(CH_2)_2-$ | $CH_3$ | H | $CH_3$ | 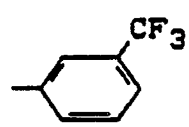 |

Tabelle 1 Fortsetzung

| $R^1$ | $R^2$ | $R^3$ | $R^4$ | 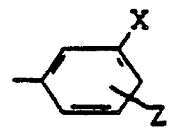 |
|---|---|---|---|---|
| $CH_3-(CH_2)_3-$ | $CH_3$ | H | $CH_3$ | 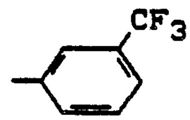 |
| 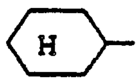 | $CH_3$ | H | $CH_3$ | 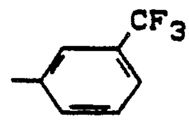 |
| 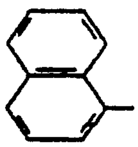 | $CH_3$ | H | $CH_3$ | 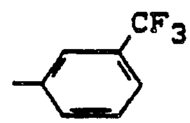 |
| 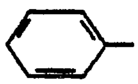 | $CH_3$ | H | H | 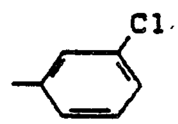 |
| 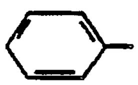 | $CH_3$ | H | $CH_3$ | 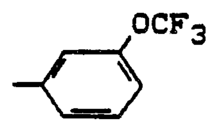 |
| 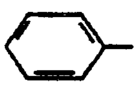 | $CH_3$ | H | $C_2H_5$ | 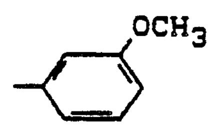 |
| 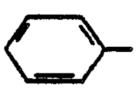 | $CH_3$ | H | $C_2H_5$ | 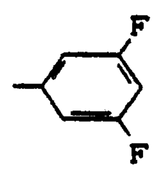 |

Tabelle 1 Fortsetzung

| $R^1$ | $R^2$ | $R^3$ | $R^4$ | 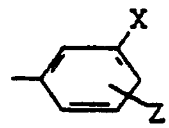 |
|---|---|---|---|---|
| 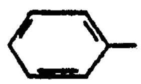 | $CH_3$ | H | $CH_3$ | 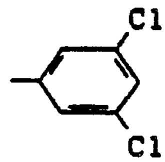 |
| 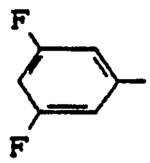 | $CH_3$ | H | $C_2H_5$ | 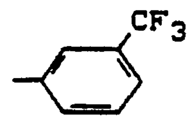 |

Verwendet man beispielsweise 3-Ethoxy-3-imino-2-(3-trifluormethyl-phenyl)-propionsäureethylester Hydrochlorid und Phenylhydrazin als Ausgangsstoffe, so läßt sich der Reaktionsablauf des erfindungsgemäßen Verfahrens (a) durch das folgende Formelschema darstellen:

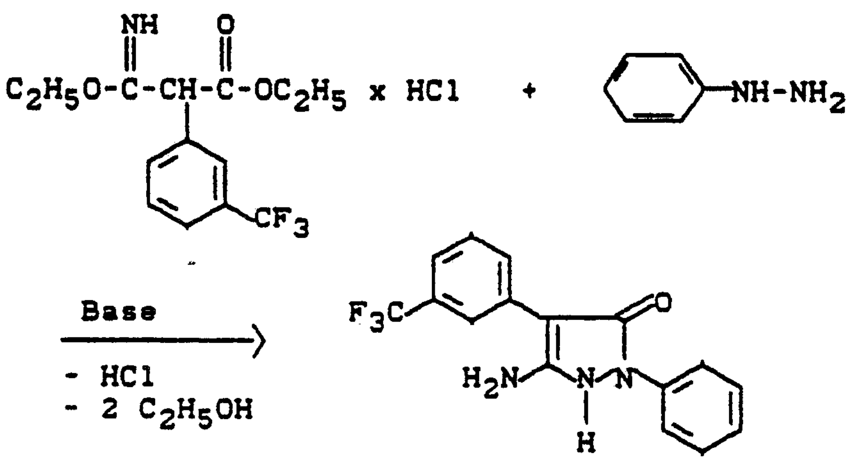


Verwendet man beispielsweise 3-Amino-1-phenyl-4-(3-trifluormethylphenyl)-2H-pyrazolin-5-on und Dimethylsulfat als Ausgangsstoffe, so läßt sich der Reaktionsablauf des erfindungsgemäßen Verfahrens (b) durch das folgende Formelschema darstellen:

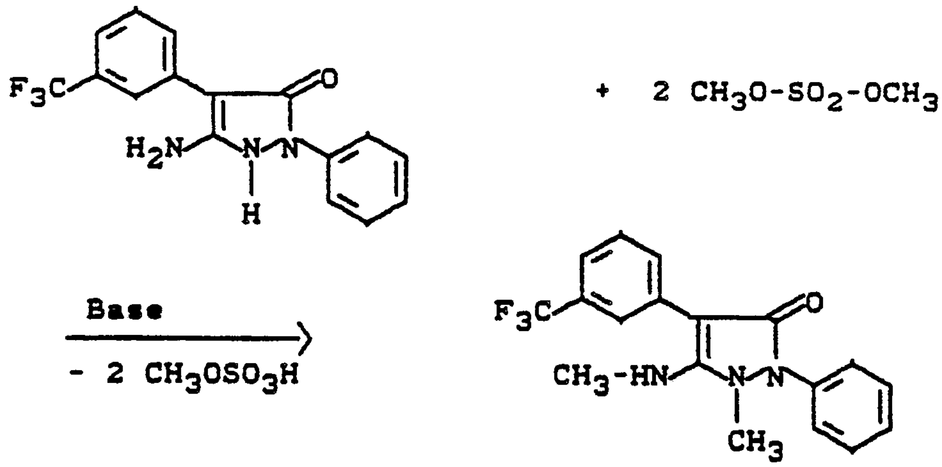


Verwendet man beispielsweise 3-Amino-1,2-dimethyl-4-(3-trifluormethylphenyl)-$\Delta^3$-pyrazolin-5-on und Allylbromid als Ausgangsstoffe, so läßt sich der Reaktionsablauf des erfindungsgemäßen Verfahrens (c) durch das folgende Formelschema darstellen:

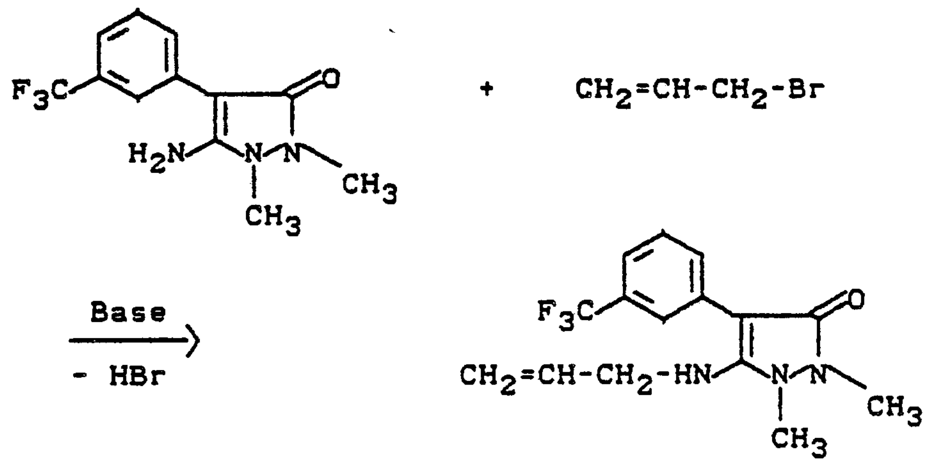


Verwendet man beispielsweise 3-Amino-1,2-dimethyl-4-(3-trifluormethylphenyl)-$\Delta^3$-pyrazolin-5-on und 1,4-Dibrombuta-1,3-dien als Ausgangsstoffe, so läßt sich der Reaktionsablauf des erfindungsgemäßen Verfahrens (d) durch das folgende Formelschema darstellen:

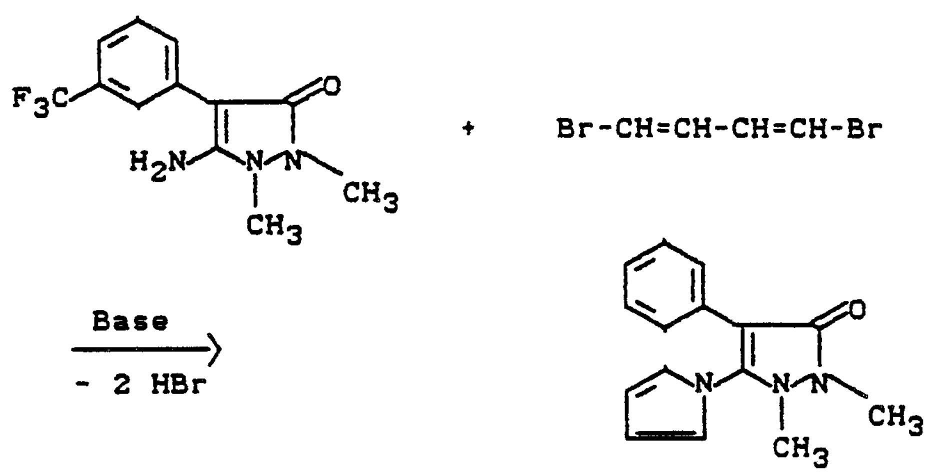


Verwendet man beispielsweise 3-Amino-2-methyl-1-phenyl-4-(3-trifluormethylphenyl)-$\Delta^3$-pyrazolin-5-on

und Acetanhydrid als Ausgangsstoffe, so läßt sich der Reaktionsablauf des erfindingsgemäßen Verfahrens (e) durch das folgende Formelschema darstellen:

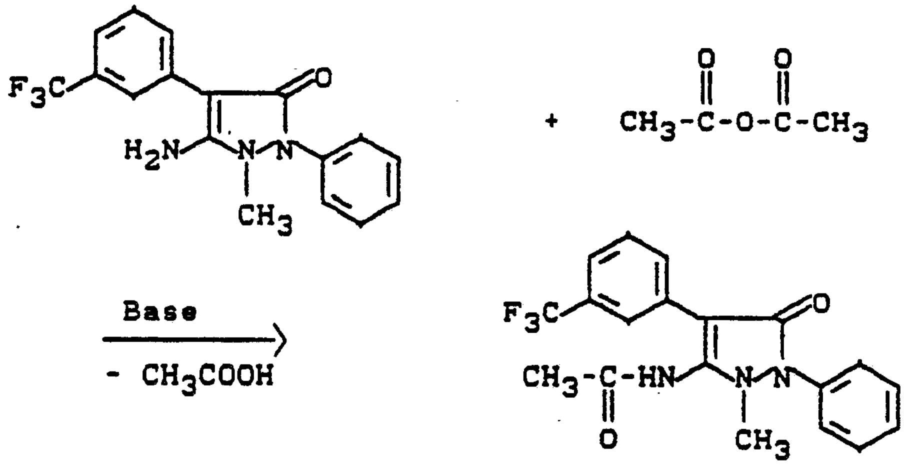


Die zur Durchführung des erfindungsgemäßen Verfahrens (a) als Ausgangsstoffe benötigten Iminomalonester-Derivate sind durch die Formel (II) allgemein definiert. In dieser Formel (II) stehen X und Z vorzugsweise für diejenigen Reste, die bereits im Zusammenhang mit der Beschreibung der erfindungsgemäßen Stoffe der Formel (I) als bevorzugt für diese Substituenten genannt wurden.

Die Iminomalonester-Derivate der Formel (II) sind noch nicht bekannt.

Man erhält sie, wenn man Phenylacetonitril-Derivate der Formel (VI),

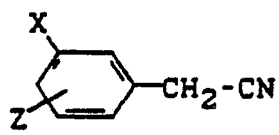 (VI)

in welcher
X und Z die oben angegebene Bedeutung haben,
zunächst in einer ersten Stufe mit Diethylcarbonat der Formel (VII)

$C_2H_5O-\overset{\overset{O}{||}}{C}-OC_2H_5$ (VII)

gegebenenfalls in Gegenwart eines Verdünnungsmittels wie beispielsweise Ethanol und gegebenenfalls in Gegenwart einer Base wie beispielsweise Natriumethylat bei Temperaturen zwischen 20 °C und 120 °C umsetzt (vgl. hierzu auch Organic Syntheses Coll. Vol. IV; S. 461 [1963]) und dann die so erhältlichen 2-Arylcyanessigester der Formel (VIII),

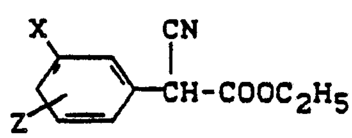 (VIII)

in welcher
X und Z die oben angegebene Bedeutung haben,
in einer 2. Stufe mit Ethanol und Chlorwasserstoff gege benenfalls in Gegenwart eines Verdünnungsmittels wie beispielsweise Diethylether bei Temperaturen zwischen -20 °C und +20 °C umsetzt (vgl. z. B. Helv. Chim. Acta 43, 1727 - 1733 [1960]).

Die Phenylacetonitrilderivate der Formel (VI) sind bekannt oder erhältlich in Analogie zu bekannten Verfahren (vgl. z. B. DE-OS 21 60 119; GB 12 38 522; DE-OS 12 00 970; US-PS 3 476 790; US-PS 3 277 106).

Diethylcarbonat der Formel (VII) ist eine allgemein bekannte Verbindung der organischen Chemie (vgl.

z. B. US-PS 3 415 867).

Die zur Durchführung des erfindungsgemäßen Verfahrens (a) weiterhin als Ausgangsstoffe benötigten Hydrazine sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Als Säureadditionssalze von Hydrazinen der Formel (III) sind Hydrochloride oder Hydroacetate besonders bevorzugt.

Die Hydrazine der Formel (III) sowie deren Säureadditionssalze sind allgemein bekannte Verbindungen der organischen Chemie.

Die zur Durchführung der erfindungsgemäßen Verfahren (b) und (d) als Ausgangsstoffe benötigten 3-Aminopyrazolin-5-one sind durch die Formel (Ia) allgemein definiert. In dieser Formel (Ia) stehen $R^1$, $R^2$, X und Z vorzugsweise für diejenigen Reste, die bereits im Zusammenhang mit der Beschreibung der erfindungsgemäßen Stoffe der Formel (I) als bevorzugt für diese Substituenten genannt wurden.

Die 3-Aminopyrazolin-5-one der Formel (Ia) sind erfindungsgemäße Verbindungen und erhältlich mit Hilfe des erfindungsgemäßen Verfahrens (a).

Die zur Durchführung des erfindungsgemäßen Verfahrens (b) weiterhin als Ausgangsstoffe benötigten Alkylierungsmittel sind durch die Formel (IVa) allgemein definiert. In dieser Formel (IVa) steht $R^5$ vorzugsweise für geradkettiges oder verzweigtes Alkyl mit 1 bis 4 Kohlenstoffatomen, insbesondere für Methyl, Ethyl sowie n-oder i-Propyl. $E^1$ steht vorzugsweise für Halogen, insbesondere für Chlor, Brom oder Iod oder für Alkoxysulfonyloxy, wie beispielsweise Methoxysulfonyloxy oder Ethoxysulfonyloxy oder für gegebenenfalls substituiertes Arylsulfonyloxy, wie beispielsweise p-Toluolsulfonyloxy.

Die Alkylierungsmittel der Formel (IVa) sind allgemein bekannte Verbindungen der organischen Chemie.

Die zur Durchführung der erfindungsgemäßen Verfahren (c) und (e) als Ausgangstoffe benötigten 3-Amino-pyrazolin-5-one sind durch die Formel (Iz) allgemein definiert.

In dieser Formel (Iz) stehen $R^1$, $R^2$, $R^3$, X und Z vorzugsweise für diejenigen Reste, die bereits im Zusammenhang mit der Beschreibung der erfindungsgemäßen Stoffe der Formel (I) als bevorzugt für diese Substituenten genannt wurden.

Die 3-Aminopyrazolin-5-one der Formel (Iz) sind erfindungsgemäße Verbindungen und erhältlich mit Hilfe der erfindungsgemäßen Verfahrens (a) und (b).

Die zur Durchführung des erfindungsgemäßen Verfahrens (c) weiterhin als Ausgangsstoffe benötigten Alkylierungsmittel sind durch die Formel (IVb) allgemein definiert. In dieser Formel (IVb) steht $R^{4-2}$ vorzugsweise für Alkenyl mit 3 bis 6 Kohlenstoffatomen, für Alkinyl mit 3 bis 6 Kohlenstoffatomen oder für Alkoxyalkyl, Alkylthioalkyl oder Alkoxycarbonylalkyl mit jeweils 1 bis 4 Kohlenstoffatomen in den einzelnen Alkylteilen; $R^{4-2}$ steht insbesondere für Allyl, Propargyl, Methoxymethyl, Methylthiomethyl, Methoxycarbonylmethyl oder Ethoxycarbonylmethyl. $E^2$ steht vorzugsweise für Halogen, insbesondere für Chlor, Brom oder Iod.

Die Alkylierungsmittel der Formel (IVb) sind allgemein bekannte Verbindungen der organischen Chemie.

Die zur Durchführung des erfindungsgemäßen Verfahrens (d) weiterhin als Ausgangsstoffe benötigten Alkylierungsmittel sind durch die Formel (IVc) allgemein definiert. In dieser Formel (IVc) steht A vorzugsweise für einen jeweils zweifach Verknüpften Alkylen- oder Alkenylenrest mit jeweils 2 bis 5 Kohlenstoffatomen, insbeson dere für einen der Reste $-CH_2CH_2-$; $-CH_2-CH_2-CH_2-CH_2-$; $-CH_2-CH_2-CH_2-CH_2-CH_2-$; $CH_2-CH=CH-CH_2-CH_2-$ oder $CH=CH--CH=CH-$. $E^3$ steht vorzugsweise für Halogen, insbesondere für Brom.

Die zur Durchführung des erfindungsgemäßen Verfahrens (e) weiterhin als Ausgangstoffe benötigten Acylierungsmittel sind durch die Formel (V) allgemein definiert. In dieser Formel (V) steht $R^{4-4}$ vorzugsweise für geradkettiges oder verzweigtes Alkanoyl mit 1 bis 5 Kohlenstoffatomen, insbesondere für Acetyl oder Propionyl. $E^4$ steht vorzugsweise für Halogen, insbesondere für Chlor oder Brom oder für einen Rest $R^{4-4}$-CO-O-, wobei $R^{4-4}$ vorzugsweise für die oben genannten Reste steht.

Die Acylierungsmittel der Formel (V) sind ebenfalls allgemein bekannte Verbindungen der organischen Chemie.

Als Verdünnungsmittel zur Durchführung des erfindungsgemäßen Verfahrens (a) kommen inerte organischen Lösungsmittel in Frage. Mit besonderem Vorzug verwendet man Alkohole, insbesondere Methanol oder Ethanol oder organische Säuren wie beispielsweise Essigsäure.

Das erfindungsgemäße Verfahren (a) wird vorzugsweise in Gegenwart eines geeigneten Reaktionshilfsmittels durchgeführt. Vorzugsweise verwendet man aprotische Basen, wobei insbesondere Alkalimetallalkoholate, wie beispielsweise Natriummethylat oder Natriumethylat in Frage kommen.

Die Reaktionstemperaturen können bei der Durchführung des erfindungsgemäßen Verfahrens (a) in einem größeren Bereich variiert werden. Im allgemeinen arbeitet man bei Temperaturen zwischen -20 °C und +50 °C, vorzugsweise bei Temperaturen zwischen 0 °C und 30 °C.

Zur Durchführung des erfindungsgemäßen Verfahrens (a) setzt man pro Mol an Iminomalonesterderivat der Formel (II) im allgemeinen 1.0 bis 1.5 Mol, vorzugsweise 1.0 bis 1.2 Mol an Hydrazin der Formel (III) und gegebenenfalls 1.0 bis 5.0 Mol, vorzugsweise 1.0 bis 3.0 Mol an Reaktionshilfsmittel ein.
Dabei ist es gegebenenfalls von Vorteil die Reaktion in Gegenwart einer trockenen Stickstoffinertgasatmosphäre durchzuführen und den pH-Wert den Reaktionsmischung konstant beim Neutralpunkt zu halten. Eventuelle Korrekturen des pH-Wertes können durch Zugabe von Alkoholat oder Eisessig vorgenommen werden.
Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung der erfindungsgemäßen Verfahren (b), (c) oder (d) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -di ethylether, Ketone wie Aceton oder Butanon, Nitrile, wie Acetonitrill oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Alkohole wie Methanol oder Ethanol.

Die erfindungsgemäßen Verfahren (b), (c) oder (d) werden vorzugsweise in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die erfindungsgemäßen Verfahren (b), (c) oder (d) können gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyldimethyl-ammoniummethylsulfat, Dimethyl-$C_{12}/C_{14}$-alkylbenzylammoniumchlcrid, Tetrabutylammoniumhydroxid, 15-Krone-5, 18-Krone-6, Triethylbenzylammoniumchlorid, Trimethylbenzylammoniumchlorid.

Die Reaktionstemperaturen können bei der Durchführung der erfindungsgemäßen Verfahren (b), (c) oder (d) in einem größeren Bereich variiert werden. Im allgemeinen arbeitet man bei Temperaturen zwischen 0 °C und 100 °C, vorzugsweise bei Temperaturen zwischen 20 °C und 80 °C.

Zur Durchführung des erfindungsgemäßen Verfahrens (b) setzt man pro Mol an 3-Amino-pyrazolin-5-on der Formel (Ia) im allgemeinen 1.0 bis 5.0 Mol, vorzugsweise 1.0 bis 3.0 Mol an Alkylierungsmittel der Formel (IVa) und gegebenenfalls 1.0 bis 5.0 Mol, vorzugsweise 1.0 bis 3.0 Mol an Reaktionshilfsmittel sowie gegebenenfalls 0.01 bis 1.0 Mol an Phasentransferkatalysator ein.

Je nach Menge des eingesetzten Alkylierungsmittel der Formel (IVa) erhält man entweder mono-, di- oder trialkylierte Verbindungen der Formel (Ib).

Je nach Art der gewählten Reaktionsbedingungen ist es möglich bei einem Unterschuß an eingesetztem Alkylierungsmittel der Formel (IVa) den Ort der Alkylierung gezielt zu beeinflussen. So wird beispielsweise durch die Wahl eines protischen Lösungsmittels bevorzugt der Ringstickstoff alkyliert, während unter wasserfreien Reaktionsbedingungen oder bei Phasentransferkatalyse eine bevorzugte Alkylierung der exocyclischen 3-Aminogruppe erfolgt. Eventuell auftretende Produktgemische lassen sich mit chromatographischen Methoden auftrennen, gegebenenfalls in Nebenreaktionen gebildete O-alkylierte Verbindungen lassen sich mit ebensolchen Methoden abtrennen.

Die Reaktionsdurchführung, Aufarbeitung und Isolierung der Reaktionsprodukte erfolgt nach allgemein üblichen Methoden (vgl. auch die Herstellungsbeispiele).

Zur Durchführung des erfindungsgemäßen Verfahrens (c) setzt man pro Mol an 3-Aminopyrazolin-5-on der Formel (Iz) im allgemeinen 1.0 bis 3.0 Mol, vorzugsweise 1.0 bis 2.0 Mol an Alkylierungsmittel der Formel (IVb) und gegenbenenfalls 1.0 bis 3.0 Mol, vorzugsweise 1.0 bis 2.0 Mol an Reaktionshilfsmittel ein.
Die Reaktionsdurchführung, Aufarbeitung und Isolierung der Reaktionsprodukte erfolgt nach allgemein üblichen Methoden.

Zur Durchführung des erfindungsgemäßen Verfahrens (d) setzt man pro Mol an 3-Aminopyrazolin-5-on der Formel (Ia) im allgemeinen 0.3 bis 0.7 Mol, vorzugsweise 0.45 bis 0.5 Mol an Alkylierungsmittel der Formel (IVc) und gegebenenfalls 1.0 bis 3.0 Mol, vorzugsweise 1.0 bis 2.0 Mol an Reaktionshilfsmittel ein.
Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahrens (e) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetra chlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester.

Das erfindungsgemäße Verfahren (e) wird vorzugsweise in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (e) in einem größeren Bereich variiert werden. Im allgemeinen arbeitet man bei Temperaturen zwischen -20 °C und +150 °C, vorzugsweise bei Temperaturen zwischen 0 °C und 120 °C.

Zur Durchführung des erfindungsgemäßen Verfahrens (e) setzt man pro Mol an 3-Aminopyrazolin-5-on der Formel (Iz) im allgemeinen 1.0 bis 3.0 Mol, vorzugsweise 1.0 bis 2.0 Mol an Acylierungsmittel der Formel (V) und gegebenenfalls 1.0 bis 3.0 Mol, vorzugsweise 1.0 bis 2.0 Mol an Reaktionshilfsmittel ein.

Die Reaktionsdurchführung, Aufarbeitung und Isolierung der Reaktionsprodukte erfolgt nach allgemein üblichen Methoden.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpul ver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage; Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser, Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wo bei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimthylethyl)-3-methylthio-1,2,4-triazin-4(4H)-on (METRIBUZIN) zur Unkrautbekämpfung in Sojabohnen, in Frage, Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch

Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

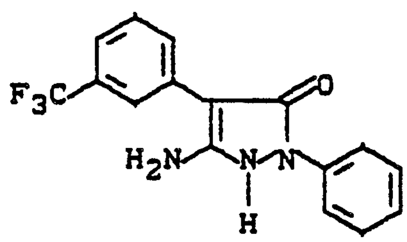

**(Verfahren (a))**

Zu 339,5 g (1.0 Mol) 2-(1-Ethoxyformimidoyl)-2-(3-trifluormethylphenyl)-essigsäureethylester Hydrochlorid in 800 ml trockenem Methanol gibt man bei 0 °C bis 5 °C unter einer trockenen Stickstoffatmosphäre tropfenweise unter Rühren innerhalb einer Stunde 59,4 g (1.1 Mol) Natriummethylat in 200 ml Methanol, anschließend ebenfalls bei 0 °C bis 5 °C tropfenweise unter Rühren innerhalb von 30 Minuten 108 g (1.0 Mol) Phenylhydrazin in 150 ml Methanol, überprüft anschließend den pH-Wert der Mischung und korrigiert falls nötig mit methanolischer Natriummethylat-Lösung oder Eisessig auf pH 7, rührt 2 Stunden bei 0 °C bis 5 °C und 16 Stunden bei Raumtemperatur, gibt dann den Reaktionsansatz in 2 l Wasser, extrahiert mit 1 l Dichlormethan und wäscht die organische Phase mit 1 l Wasser, wobei das Produkt aus der organischen Phase ausfällt. Man trennt die Phasen, saugt ab, wäscht mit 100 ml Dichlormethan nach und trocknet den so erhaltenen Feststoff.

Man erhält 268 g (84 % der Theorie) an 3-Amino-1-phenyl-4-(3-trifluormethylphenyl)-2H(4H)-pyrazolin-5-on vom Schmelzpunkt 187 °C.

Herstellung der Ausgangsverbindung

Beispiel (II-1)

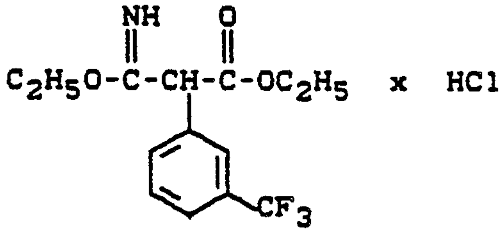

Zu 257 g (1.0 Mol) 2-(3-Trifluormethylphenyl)-cyanessigsäureethylester in 1 l Diethylether gibt man bei

0 °C bis 5 °C unter einer trockenen Stickstoffatmosphäre 46 g (1.0 Mol) Ethanol, leitet anschließend bis zur Sättigung trockenes Chlorwasserstoffgas ein, rührt weitere 30 Minuten bei 0 °C bis 5 °C, engt im Vakuum ein, verrührt den Rückstand mit Ether und saugt ab.

Man erhält 340 g (100 % der Theorie) an 2-(1-Ethoxyformimidoyl)-2-(3-trifluormethylphenyl)-essigsäureethylester Hydrochlorid, welches ohne zusätzliche Reinigung sofort weiter umgesetzt wird.

Beispiel (VIII-1)

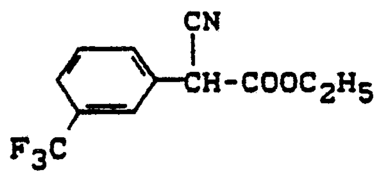

Eine Lösung von 23,9 g (1.04 Mol) Natrium in 600 ml Ethanol wird bei 70 °C unter einer trockenen Stickstoffatmosphäre mit 600 ml (5.0 Mol) Kohlensäurediethylester und 185 g (1.0 Mol) 3-Trifluormethylbenzylcyanid versetzt. Unter vermindertem Druck wird anschließend das Ethanol abdestilliert und gleichzeitig 160 ml absolutes Toluol zugetropft. Nach beendeter Destillation gibt man 600 ml Wasser zu, säuert mit 80 ml Eisessig an, trennt die organische Phase ab, wäscht mit Wasser, trocknet über Natriumsulfat, engt im Vakuum ein und destilliert den Rückstand im Hochvakuum.

Man erhält 228 g (88,7 % der Theorie) an 2-(3-Trifluormethylphenyl)-cyanessigsäureethylester vom Siedepunkt 105 °C bis 111 °C bei 0.1 mbar.

Beispiel 2

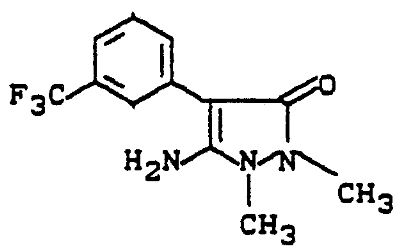

**(Verfahren (a))**

Zu 133 g (1.0 Mol) N,N′-Dimethylhydrazin Dihydrochlorid in 250 ml Methanol gibt man 108 g (2.0 Mol) Natriummethylat in 250 ml Methanol, rührt 30 Minuten bei Raumtemperatur, saugt ab und gibt das Filtrat tropfenweise unter einer trockenen Stickstoffatmosphäre bei 0 °C bis 5 °C unter Rühren und Kühlung in eine Lösung von 339,5 g (1.0 Mol) 2-(1-Ethoxyformimidoyl)-2-(3-trifluormethylphenyl)-essigsäureethylester Hydrochlorid in 500 ml Methanol, zu der man vorher schon ebenfalls bei 0 °C bis 5 °C unter einer trockenen Stickstoffatmosphäre tropfenweise unter Rühren innerhalb von einer Stunde 59,4 g (1.1 Mol) Natriummethylat in 200 ml Methanol gegeben hat. Nach beendeter Zugabe stellt man mit Natriummethylat oder Eisessig den pH-Wert auf den Neutralpunkt ein, rührt 4 Stunden bei 0 °C bis 5 °C und anschließend 16 Stunden bei Raumtemperatur, gibt dann den Reaktionsansatz in 2 000 ml Wasser, extrahiert mit 600 ml Dichlormethan, trocknet die organische Phase über Natriumsulfat und engt im Vakuum ein. Der Rückstand wird mit Dichlormethan/Petrolether (1 : 1) verrührt, abgesaugt und getrocknet.

Man erhält 81 g (30 % der Theorie) an 3-Amino-1,2-dimethyl-4-(3-trifluormethylphenyl)-$\Delta^3$-pyrazolin-5-on vom Schmelzpunkt 156 °C - 157 °C.

Beispiel 3

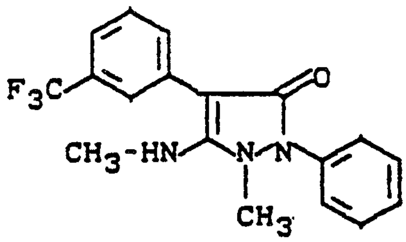

(Verfahren (b))

Zu 15,95 g (0.05 Mol) 3-Amino-1-phenyl-4-(3-trifluorme thylphenyl)-2H(4H)-pyrazolin-5-on in 375 ml Dichlormethan gibt man 15 ml 45 %ige wäßrige Natronlauge und 0,25 g Tributylbenzylammoniumbromid und dann unter Rühren bei Raumtemperatur tropfenweise 6,3 g (0.05 Mol) Dimethylsulfat in 50 ml Dichlormethan. Nach beendeter Zugabe rührt man 16 Stunden bei Raumtemperatur, gibt dann 250 ml Wasser zu, trennt die organische Phase ab, wäscht mit Wasser, trocknet über Natriumsulfat, engt im Vakuum ein und reinigt den Rückstand durch Chromatographie an Kieselgel (Laufmittel : Petrolether/Essigester 7 : 3).

Man erhält 5,0 g (30 % der Theorie) an 2-Methyl-3-methylamino-1-phenyl-4-(3-trifluormethylphenyl)-$\Delta^3$-pyrazolin-5-on vom Schmelzpunkt 205 °C bis 206 °C.

Beispiel 4

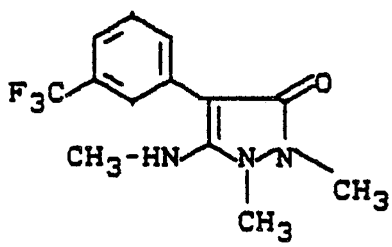

(Verfahren (b))

Zu 2,71 g (0.01 Mol) 3-Amino-1,2-dimethyl-4-(3-trifluormethylphenyl-$\Delta^3$-pyrazolin-5-on in 75 ml Dichlormethan gibt man 3 ml 45 %ige wäßrige Natronlauge und 0,05 g Tributylbenzylammoniumbromid und anschließend unter Rühren bei Raumtemperatur tropfenweise 1,26 g (0.01 Mol) Dimethylsulfat in 10 ml Dichlormethan, rührt nach beendeter Zugabe 16 Stunden bei Raumtemperatur, gibt 10 ml Wasser zu, trennt die organische Phase ab, wäscht mit Wasser, trocknet über Natriumsulfat, engt im Vakuum ein und reinigt den Rückstand durch Chromatographie an Kieselgel (Laufmittel: Dichlormethan/Methanol 40 : 1).

Man erhält 1,4 g (49 % der Theorie) an 1,2-Dimethyl-3-methylamino-4-(3-trifluormethylphenyl-$\Delta^3$-pyrazolin-5-on vom Schmelzpunkt 152 °C - 153 °C.

Beispiel 5

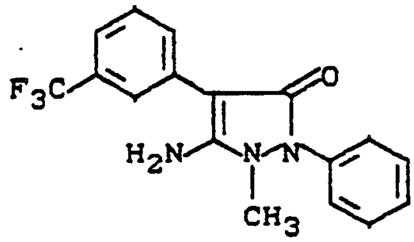

(Verfahren (b))

Zu 8,0 g (0.1 Mol) 45 %iger wäßriger Natronlauge und 32,0 g (0.1 Mol) 3-Amino-1-phenyl-4-(3-trifluormethylphenyl)-2H(4H)-pyrazolin-5-on in 50 ml Methanol gibt man bei Rückflußtemperatur tropfenweise unter Rühren 12,5 g (0.1 Mol) Dimethylsulfat, läßt anschließend den Ansatz unter Rühren auf Raumtemperatur kommen, engt im Vakuum ein, verrührt den Rückstand mit warmem Wasser, saugt ab, verrührt den Rückstand mit 30 ml Dichlormethan, saugt wieder ab und trocknet.

Man erhält 13 g (40 % der Theorie) an 3-Amino-2-methyl-1-phenyl-4-(3-trifluormethylphenyl)-$\Delta^3$-pyrazolin-5-on vom Schmelzpunkt >250 °C.

In entsprechender Weise und gemäß den allgemeinen Angaben zur Herstellung erhält man die folgenden 3-Aminopyrazolin-5-one der allgemeinen Formel (I):

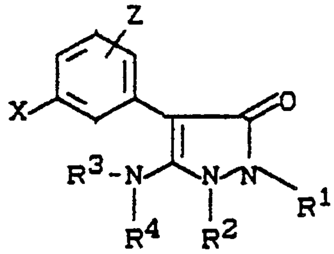

(I)

<u>Tabelle 2</u>

| Beisp.-Nr. | $R^1$ | $R^2$ | $R^3$ | $R^4$ | 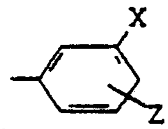 | Schmelzpunkt (°C) |
|---|---|---|---|---|---|---|
| 6 | 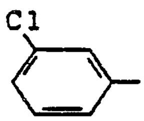 | H | H | H | 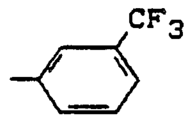 | 193 - 194 |
| 7 | 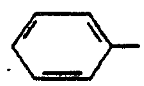 | $CH_3$ | $CH_3$ | $CH_3$ | 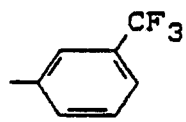 | 167 - 168 |
| 8 | $CH_3$ | $CH_3$ | $CH_3$ | $CH_3$ | 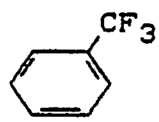 | 93 - 94 |
| 9 | 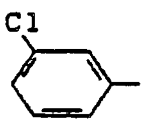 | $CH_3$ | H | $CH_3$ | 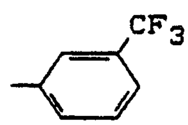 | 205-206 |
| 10 | 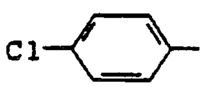 | $CH_3$ | H | H | 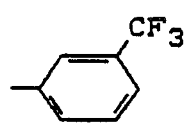 | >250 |

Tabelle 2 (Fortsetzung)

| Beisp.-Nr. | $R^1$ | $R^2$ | $R^3$ | $R^4$ | 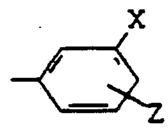 | Schmelzpunkt (°C) |
|---|---|---|---|---|---|---|
| 11 | $CH_3$ | $CH_3$ | H | $\overset{O}{\overset{\parallel}{-C}}-CH_3$ | 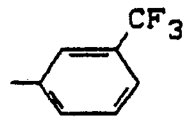 | 185-186 |
| 12 | 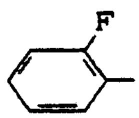 | H | H | H | 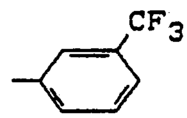 | 219 |
| 13 | 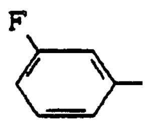 | H | H | H | 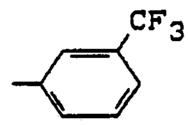 | 198 |
| 14 | 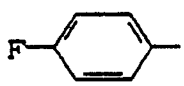 | H | H | H | 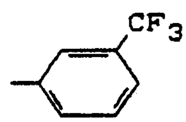 | 164 |
| 15 | 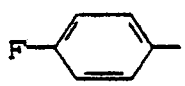 | $CH_3$ | H | $CH_3$ | 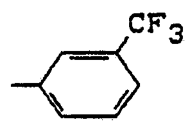 | 195-196 |
| 16 | 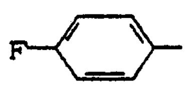 | $CH_3$ | $CH_3$ | $CH_3$ | 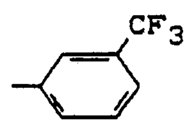 | 195-196 |
| 17 | 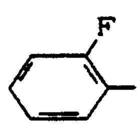 | $CH_3$ | H | H | 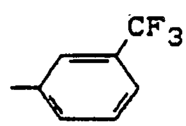 | 230-231 |
| 18 | 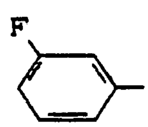 | $CH_3$ | H | H | 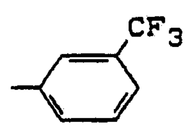 | 244-246 |
| 19 | 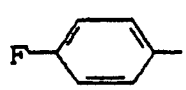 | $CH_3$ | H | H | 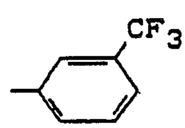 | >250 |

Tabelle 2 (Fortsetzung)

| Beisp.-Nr. | $R^1$ | $R^2$ | $R^3$ | $R^4$ | 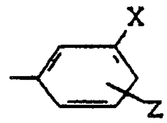 | Schmelzpunkt (°C) |
|---|---|---|---|---|---|---|
| 20 | 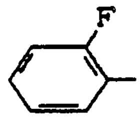 | $CH_3$ | H | $CH_3$ | 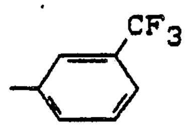 | 187 |
| 21 | 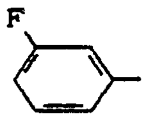 | $CH_3$ | H | $CH_3$ | 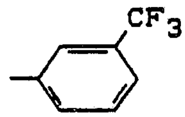 | 191 |
| 22 | 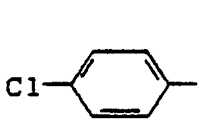 | $CH_3$ | $CH_3$ | $CH_3$ | 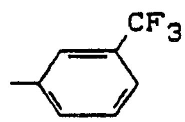 | 156 |
| 23 | 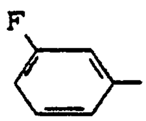 | $CH_3$ | $CH_3$ | $CH_3$ | 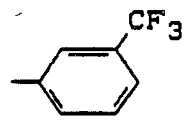 | 149 |
| 24 | 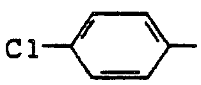 | $CH_3$ | H | $CH_3$ | 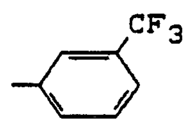 | 157 |
| 25 | 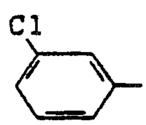 | $CH_3$ | H | H | 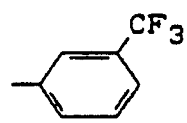 | 235 |
| 26 | 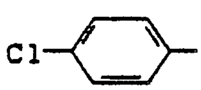 | H | H | H | 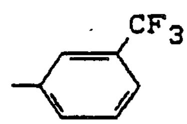 | 178-179 |

Anwendungsbeispiele

In dem folgenden Anwendungsbeispiel wurde die nachstehend aufgeführte Verbindung als Vergleichsubstanz eingesetzt:

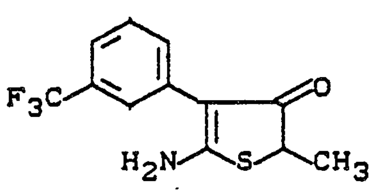

(A)

2-Amino-5-methyl-3-(3-trifluormethyl-phenyl)-5H-thiazolin-4-on
(bekannt aus US-PS 4 596 595/Beispiel 3).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z. B. die Verbindung gemäß Herstellungsbeispielen: (3), (9), (15), (20), (21), (22).

**Ansprüche**

1. 3-Aminopyrazolin-5-one der Formel (I),

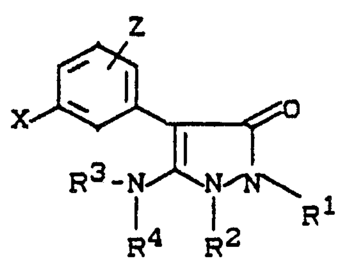

(I)

in welcher
$R^1$ für Alkyl, Alkenyl, Halogenalkyl, Halogenalkenyl, Alkoxyalkyl, Alkylthioalkyl, Cycloalkyl, für gegebenenfalls substituiertes Aralkyl oder für gegebenenfalls substituiertes Aryl steht,
$R^2$ für Wasserstoff oder Alkyl steht,
$R^3$ für Wasserstoff oder Alkyl steht,
$R^4$ für Wasserstoff, Alkyl, Alkenyl, Alkinyl, Alkoxyalkyl, Alkylthioalkyl, Alkoxycarbonylalkyl oder Alkanoyl steht oder
$R^3$ und $R^4$ gemeinsam für einen jeweils zweifach verknüpften Alkylen- oder Alkenylenrest stehen,
X für Halogen, Alkyl, Alkoxy, Alkylthio, Halogenalkyl, Halogenalkoxy oder Halogenalkylthio steht und
Z für Wasserstoff, Halogen, Alkyl, Alkoxy, Alkylthio, Halogenalkyl, Halogenalkoxy oder Halogenalkylthio steht,
sowie deren Säureadditionssalze.

2. 3-Aminopyrazolin-5-one der Formel (I) gemäß Anspruch 1,
in welcher
$R^1$ für jeweils geradkettiges oder verzweigtes Alkyl mit 1 bis 6 Kohlenstoffatomen, Alkenyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und 1 bis 5 gleichen oder verschiedenen Halogenatomen, Alkoxyalkyl mit jeweils 1 bis 4 Kohlenstoffatomen in den einzelnen Alkylteilen oder Alkylthioalkyl mit jeweils 1 bis 4 Kohlenstoffatomen in den einzelnen Alkylteilen, für Cycloalkyl mit 3 bis 7 Kohlenstoffatomen oder für jeweils im Arylteil gegebenenfalls einfach oder mehrfach, gleich oder verschieden substituiertes Aralkyl mit 1 bis 4 Kohlenstoffatomen im Alkylteil und 6 bis 10 Kohlenstoffaotmen im Arylteil oder Aryl mit 6 bis 10 Kohlenstoffatomen steht, wobei als Arylsubstituierten jeweils in Frage

kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl,

$R^2$ für Wasserstoff oder für geradkettiges oder verzweigtes Alkyl mit 1 bis 4 Kohlenstoffatomen steht,

$R^3$ für Wasserstoff oder für geradkettiges oder verzweigtes Alkyl mit 1 bis 4 Kohlenstoffatomen steht,

$R^4$ für Wasserstoff oder für jeweils geradkettiges oder verzweigtes Alkyl mit 1 bis 4 Kohlenstoffatomen, Alkenyl mit 3 bis 6 Kohlenstoffatomen, Alkinyl mit 3 bis 6 Kohlenstoffatomen, Alkoxyalkyl, Alkylthioalkyl oder Alkoxycar bonylalkyl mit jeweils 1 bis 4 Kohlenstoffatomen in den einzelnen Alkylteilen oder Alkanoyl mit 1 bis 5 Kohlenstoffatomen steht oder

$R^3$ und $R^4$ gemeinsam für einen jeweils zweifach verknüpften Alkylen- oder Alkenylenrest mit jeweils 2 bis 5 Kohlenstoffatomen stehen,

X für Fluor, Chlor, Brom, Iod, für jeweils geradkettiges oder verzweigtes Alkyl, Alkoxy oder Alkylthio mit jeweils 1 bis 4 Kohlenstoffatomen oder für jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen steht und

Z für Wasserstoff, Fluor, Chlor, Brom, Iod, für jeweils geradkettiges oder verzweigtes Alkyl, Alkoxy oder Alkylthio mit jeweils 1 bis 4 Kohlenstoffatomen oder für jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen steht, sowie deren Säureadditionssalze.

3. 3-Aminopyrazolin-5-one der Formel (I) gemäß Anspruch 1,

in welcher

$R^1$ für Methyl, Ethyl, n- oder i-Propyl, n-, i-, s-oder t-Butyl, für Allyl, n- oder i-Butenyl, für Halogenalkyl mit 1 bis 2 Kohlenstoffatomen und 1 bis 5 gleichen oder verschiedenen Halogenatomen, für jeweils gegebenenfalls einfach oder zweifach durch Chlor substituiertes Allyl, n-Butenyl oder i-Butenyl, für Methoxymethyl, Methylthiomethyl, für Cyclopropyl, Cyclopentyl, Cyclohexyl oder für jeweils gegebenenfalls ein- bis dreifach gleich oder verschieden substituiertes Phenyl, Naphthyl, Indenyl, Benzyl oder Phenylethyl steht, wobei als Arylsubstituenten jeweils in Frage kommen: Fluor, Chlor, Brom, Cyano, Nitro, Methyl, Ethyl, n-oder i-Propyl, n-, i-, s-oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder gegebenenfalls ein- bis dreifach, gleich oder verschieden durch Fluor, Chlor, Brom, Methyl und/oder Ethyl substituiertes Phenyl,

$R^2$ für Wasserstoff, Methyl oder Ethyl steht,

$R^3$ für Wasserstoff, Methyl, Ethyl, n- oder i-Propyl, n-, i-, s- oder t-Butyl steht,

$R^4$ für Wasserstoff, Methyl, Ethyl, n- oder i-Propyl, n-, i-, s- oder t-Butyl, Allyl, Propargyl, Methoxymethyl, Methylthiomethyl, Methoxycarbonylmethyl, Ethoxycarbonylmethyl, für Acetyl oder für Propionyl steht oder

$R^3$ und $R^4$ gemeinsam mit dem Stickstoffatom, an welches sie gebunden sind für einen der - Reste

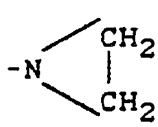 ; 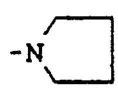 ; 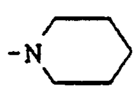 ; 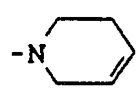 oder

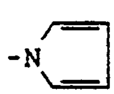 stehen,

X für Fluor, Chlor, Brom, Methyl, Methoxy, Methylthio, Trifluormethyl, Trifluormethoxy oder Trifluormethylthio steht und

Z für Wasserstoff, Fluor, Chlor, Brom, Methyl, Methoxy, Methylthio, Trifluormethyl, Trifluormethoxy oder Trifluormethylthio steht.

4. Verfahren zur Herstellung von 3-Aminopyrazolin-5-onen der Formel (I),

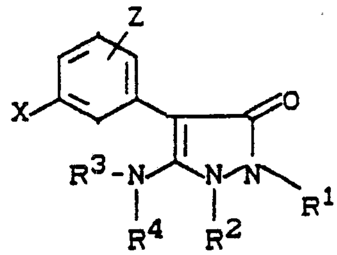

(I)

in welcher
$R^1$ für Alkyl, Alkenyl, Halogenalkyl, Halogenalkenyl, Alkoxyalkyl, Alkylthioalkyl, Cycloalkyl, für gegebenenfalls substituiertes Aralkyl oder für gegebenenfalls substituiertes Aryl steht,
$R^2$ für Wasserstoff oder Alkyl steht,
$R^3$ für Wasserstoff oder Alkyl steht,
$R^4$ für Wasserstoff, Alkyl, Alkenyl, Alkinyl, Alkoxyalkyl, Alkylthioalkyl, Alkoxycarbonylalkyl oder Alkanoyl steht oder
$R^3$ und $R^4$ gemeinsam für einen jeweils zweifach verknüpften Alkylen- oder Alkenylenrest stehen,
X für Halogen, Alkyl, Alkoxy, Alkylthio, Halogenalkyl, Halogenalkoxy oder Halogenalkylthio steht und
Z für Wasserstoff, Halogen, Alkyl, Alkoxy, Alkylthio, Halogenalkyl, Halogenalkoxy oder Halogenalkylthio steht,
sowie deren Säureadditionssalze,
dadurch gekennzeichnet, daß man
(a) 3-Aminopyrazolin-5-one der Formel (Ia),

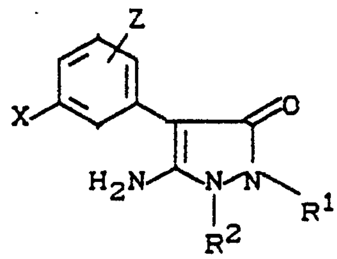

(Ia)

in welcher
$R^1$, $R^2$, X und Z die oben angegebene Bedeutung haben,
erhält, wenn man Iminomalonesterderivate der Formel (II),

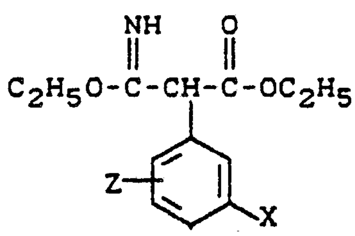

x HCl (II)

in welcher
X und Z die oben angegebene Bedeutung haben,
mit Hydrazinen der Formel (III),
$R^1 - NH - NH - R^2$ (III)
in welcher
$R^1$ und $R^2$ die oben angegebene Bedeutung haben,
oder deren Säureadditionssalzen gegebenenfalls in Gegenwart eines Verdünnungsmittels und gegebenenfalls in Gegenwart eines Reaktionshilfsmittels umsetzt; oder daß man
(b) 3-Aminopyrazolin-5-one der Formel (Ib),

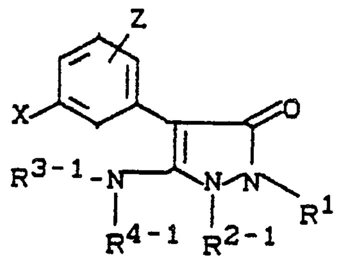

(Ib)

in welcher mindestens einer der Reste $R^{2-1}$, $R^{3-1}$ oder $R^{4-1}$ für Alkyl steht und gleichzeitig die beiden nicht betroffenen Reste jeweils für Wasserstoff oder Alkyl stehen und
$R^1$, X und Z die oben angegebene Bedeutung haben,
erhält, wenn man die nach Verfahren (a) erhältlichen 3-Aminopyrazolin-5-one der Formel (Ia),

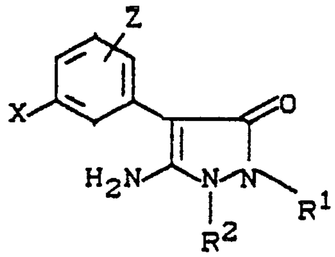

(Ia)

in welcher
$R^1$, $R^2$, X und Z die oben angegebene Bedeutung haben,
mit Alkylierungsmitteln der Formel (IVa),
$R^5$ - $E^1$ (IVa)
in welcher
$R^5$ für Alkyl steht und
$E^1$ für eine elektronenanziehende Abgangsgruppe steht,
gegebenenfalls in Gegenwart eines Verdünnungsmittels und gegebenenfalls in Gegenwart eines Reaktionshilfsmittels sowie gegebenenfalls in Gegenwart eines Phasentransferkatalysators umsetzt; oder daß man
(c) 3-Aminopyrazolin-5-one der Formel(Ic),

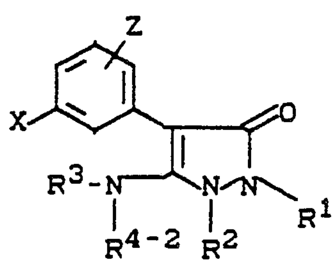

(Ic)

in welcher
$R^{4-2}$ für Alkenyl, Alkinyl, Alkoxyalkyl, Alkylthioalkyl oder Alkoxycarbonylalkyl steht und
$R^1$, $R^2$, $R^3$, X und Z die oben angegebene Bedeutung haben,
erhält, wenn man die nach Verfahren (a) und (b) erhältlichen 3-Aminopyrazolin-5-one der Formel (Iz),

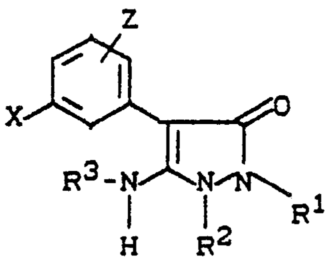

(Iz)

in welcher
$R^1$, $R^2$, $R^3$, X und Z die oben angegebene Bedeutung haben,
mit Alkylierungsmitteln der Formel (IVb),
$R^{4-2}$ - $E^2$ (IVb)
in welcher
$R^{4-2}$ die oben angegebene Bedeutung hat und
$E^2$ für eine elektronenanziehende Abgangsgruppe steht,
gegebenenfalls in Gegenwart eines Verdünnungsmittels und gegebenenfalls in Gegenwart eines Reaktionshilfsmittels sowie gegebenenfalls in Gegenwart eines Phasentransferkatalysators umsetzt; oder daß man
(d) 3-Aminopyrazolin-5-one der Formel (Id),

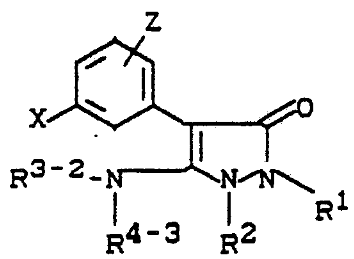

(Id)

in welcher
$R^{3-2}$ und $R^{4-3}$ gemeinsam für einen jeweils zweifach verknüpften Alkylen- oder Alkenylenrest stehen und
$R^1$, $R^2$, X und Z die oben angegebene Bedeutung haben,
erhält, wenn man die nach Verfahren (a) erhältlichen 3-Aminopyrazolin-5-one der Formel (Ia),

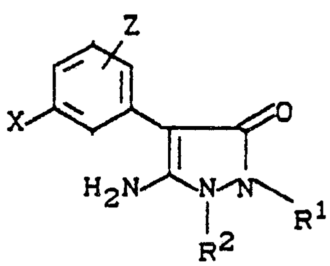

(Ia)

in welcher
$R^1$, $R^2$, X und Z die oben angegebene Bedeutung haben,
mit Alkylierungsmitteln der Formel (IVc),
$E^3$ - A - $E^3$ (IVc)
in welcher
A für einen Alkylen- oder Alkenylenrest steht und
$E^3$ für eine elektronenanziehende Abgangsgruppe steht,
gegebenenfalls in Gegenwart eines Verdünnungsmittels und gegebenenfalls in Gegenwart eines Reaktionshilfsmittels sowie gegebenenfalls in Gegenwart eines Phasentransferkatalysators umsetzt; oder daß man
(e) 3-Aminopyrazolin-5-one der Formel (Ie),

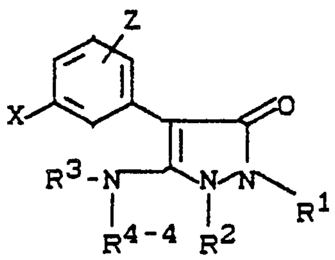

(Ie)

in welcher
$R^{4-4}$ für Alkanoyl steht und
$R^1$, $R^2$, $R^3$, X und Z die oben angegebene Bedeutung haben,
erhält, wenn man die nach Verfahren (a) oder (b) erhältlichen 3-Aminopyrazolin-5-one der Formel (Iz),

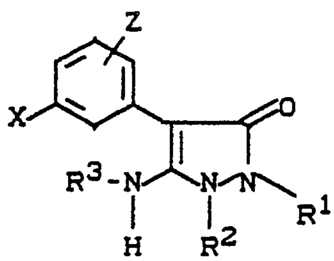

(Iz)

in welcher
$R^1$, $R^2$, $R^3$, X und Z die oben angegebene Bedeutung haben,
mit Acylierungsmitteln der Formel (V),
$R^{4-4}$ - $E^4$ (V)
in welcher
$R^{4-4}$ die oben angegebene Bedeutung hat und
$E^4$ für eine elektronenanziehende Abgangsgruppe steht,
gegebenenfalls in Gegenwart eines Verdünnungsmittels und gegebenenfalls in Gegenwart eines Reaktionshilfsmittels umsetzt;
und gegebenenfalls anschließend eine Säure addiert.

5. Herbizide Mittel, gekennzeichnet durch einen Gehalt an mindestens einem 3-Aminopyrazolin-5-on der Formel (I) gemäß den Ansprüchen 1 bis 4.

6. Verfahren zur Bekämpfung von Unkräutern, dadurch gekennzeichnet, daß man 3-Aminopyrazolin-5-one der Formel (I) gemäß den Ansprüchen 1 bis 4 auf die Unkräuter und/oder ihren Lebensraum einwirken läßt.

7. Verwendung von 3-Aminopyrazolin-5-onen der Formel (I) gemäß den Ansprüchen 1 bis 4 zur Bekämpfung von Unkräutern.

8. Verfahren zur Herstellung von herbiziden Mitteln, dadurch gekennzeichnet, daß man 3-Aminopyrazolin-5-one der Formel (I) gemäß den Ansprüchen 1 bis 4 mit Streckmitteln und/oder oberflächenaktiven Substanzen vermischt.

9. Iminomalonesterderivate der Formel (II),

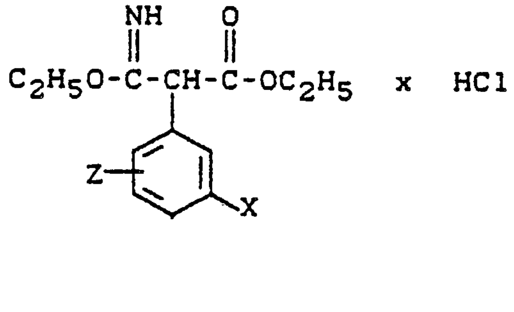

(II)

in welcher

X für Halogen, Alkyl, Alkoxy, Alkylthio, Halogenalkyl, Halogenalkoxy oder Halogenalkylthio steht und
Z für Wasserstoff, Halogen, Alkyl, Alkoxy, Alkylthio, Halogenalkyl, Halogenalkoxy oder Halogenalkylthio steht.

10. Verfahren zur Herstellung von Iminomalonesterderivaten der Formel (II),

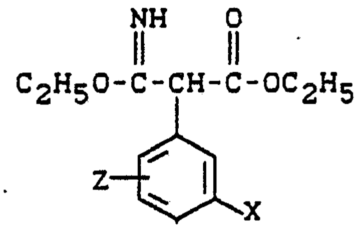

x HCl (II)

in welcher
X für Halogen, Alkyl, Alkoxy, Alkylthio, Halogenalkyl, Halogenalkoxy oder Halogenalkylthio steht und
Z für Wasserstoff, Halogen, Alkyl, Alkoxy, Alkylthio, Halogenalkyl, Halogenalkoxy oder Halogenalkylthio steht,
dadurch gekennzeichnet, daß man Phenylacetonitril-Derivate der Formel (VI),

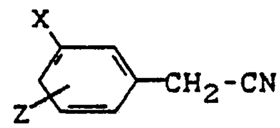

(VI)

in welcher
X und Z die oben angegebene Bedeutung haben,
zunächst in einer ersten Stufe mit Diethylcarbonat der Formel (VII)

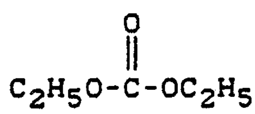

(VII)

gegebenenfalls in Gegenwart eines Verdünnungsmittels und gegebenenfalls in Gegenwart einer Base umsetzt und dann die so erhältlichen 2-Arylcyanessigester der Formel (VIII),

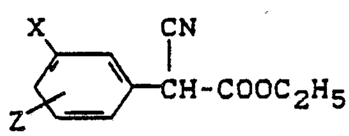

(VIII)

in welcher
X und Z die oben angegebene Bedeutung haben,
in einer 2. Stufe mit Ethanol und Chlorwasserstoff gegebenenfalls in Gegenwart eines Verdünnungsmittels umsetzt.

Europäisches Patentamt

# EUROPÄISCHER RECHERCHENBERICHT

Nummer der Anmeldung

EP 88 11 8631

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 180 707 (BAYER AG) --- | | C 07 D 231/52<br>A 01 N 43/56 |
| A | EP-A-0 076 212 (EASTMAN KODAK CO.) --- | | |
| D,A | US-A-4 596 595 (CHEVRON RESEARCH CO.) ----- | | |
| | | | **RECHERCHIERTE SACHGEBIETE (Int. Cl.4)** |
| | | | C 07 D 231/00<br>A 01 N 43/00 |

**Der vorliegende Recherchenbericht wurde für alle Patentansprüche erstellt**

| Recherchenort | Abschlußdatum der Recherche | Prüfer |
|---|---|---|
| DEN HAAG | 10-02-1989 | DE BUYSER I.A.F. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)